# EUROPEAN PATENT APPLICATION

(11) **EP 2 151 503 A1**
(43) Date of publication of application: **10.02.2010**
(21) Application number: 08161325.9
(22) Date of filing: 29.07.2008
(51) Int. Cl.: C12Q 1/68

(54) **Method for uniformly labelled ligation detection reactions (LDRs) associated to universal arrays and labelled probes thus obtained**

(71) Applicant: Fondazione Parco Tecnologico Padano, 26900 Lodi Lo (IT)
(72) Inventor: Caetano Rodrigues, Alexandre, 71540-135 Brasilia D.F. (BR); Lauri, Andrea, 26900 Lodi LO (IT); Mariani, Paola Ornella, 20141 Milano MI (IT)
(74) Representative: Predazzi, Valentina

(57) **Abstract**

The invention relates to a method to circumvent the natural loss of fluorescence of fluorophores used to label LDR probes due to freezing/thawing cycles which often results in inconsistent and biased experiment outputs; the method is based on indirect labeling of LDR probes by the addition of universal sequences (ULS) which hybridize to fluorophore-labeled oligonucleotides with complementary universal sequences (cULS). The invention also relates to LDR DPs for LDR-UA assays, comprising ULS and labelled cULS.

## Description

### SUMMARY OF THE INVENTION

The present description relates to a method to circumvent the natural loss of fluorescence of fluorophores used to label LDR probes due to freezing/thawing cycles which often results in inconsistent and biased experiment outputs; the method is based on indirect labeling of LDR probes by the addition of universal sequences (ULS) which hybridize to fluorophore-labeled oligonucleotides with complementary universal sequences (cULS). The invention also relates to LDR DPs for LDR-UA assays, comprising ULS and labelled cULS.

### BACKGROUND OF THE INVENTION

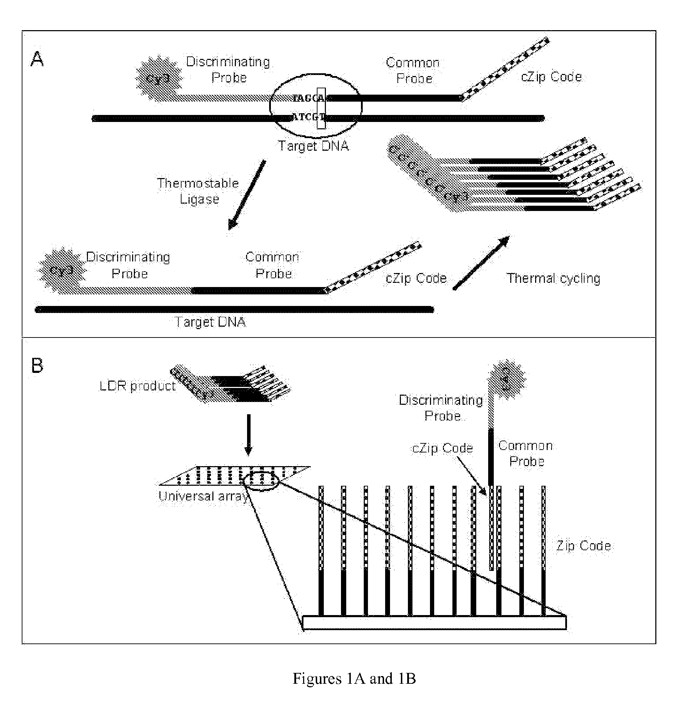

The Ligation Detection Reaction - Universal Array (LDR-UA) technique (Gerry, N. P., N. E. Witowski, J. Day, R. P. Hammer, G. Barany, and F. Barany, Universal DNA microarray method for multiplex detection of low abundance point mutations J. Mol. Biol. (1999) 292:251-262; US patent US06852487) allows the detection of Single Nucleotide Polymorphisms (SNPs) on DNA molecules. The LDR-UA technique takes advantage of two different probes, called Common Probe (CP) and Discriminating Probe (DP), that are designed to anneal juxtaposed on target single strand DNA. DP anneals on the DNA at the 5' end of CP. The two probes can be ligated by a thermo-stable ligase such as the Pfu Ligase. If the complementarity between the 3' end of the DP and the target DNA is not perfect, the ligation reaction is compromised (Figure 1A). For this reason the last base at the 3' end of the DP is also called discriminating base.

The system requires that the probes carry the following modifications:
- Each DP must be labelled with a distinct fluorophore at its 5' end.
- The CP must be phosphorylated at its 5' end and must be extended at its 3' end with a "cZIP Code" sequence, that is the complementary and inverse of the "Zip Code" sequence spotted on the Universal Array. Every CP corresponds to a different "ZIP Code".

The ligation product is hybridized to the UA. Each UA spot is composed of different "Zip Code" DNA sequences that capture the corresponding "cZIP Code" sequences at the CP 3' end. The CP-DP ligation event positions the fluorophore at the 5' end of the DP on the corresponding UA spot, that is visualized by the subsequent scanning of the UA (Figure 1B). The signal from a spot therefore indicates the perfect match between the DP and the target DNA.

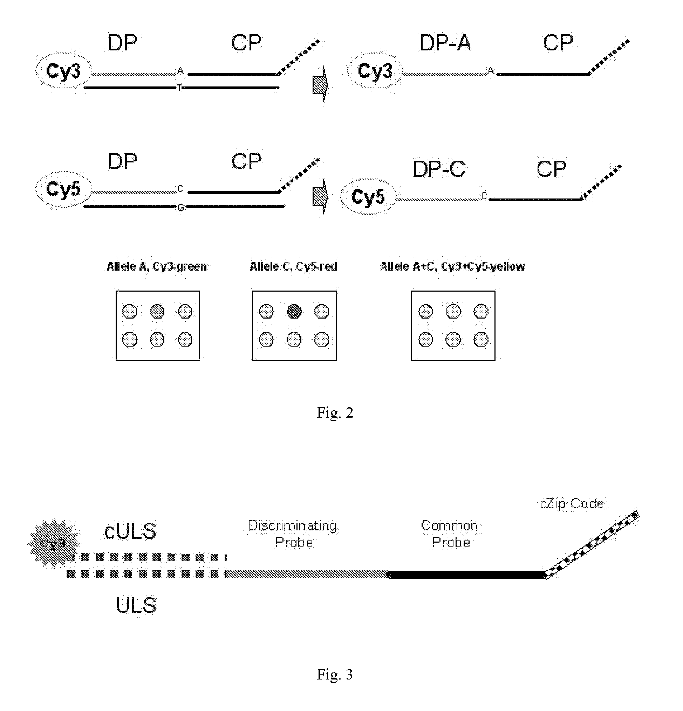

Genotyping experiments aim to identify which nucleotide is present at a given position on a DNA sequence. The possibility to identify more than one nucleotide per position (e.g. in the case of polymorphic sequences representing more than one allele) is crucially important. The LDR-UA technique offers the chance to determine which nucleotides are present at the 3' end of the DP, by implementing DPs with different discriminating nucleotides and associated to fluorophores of different colours (Figure 2).

The detection of polymorphisms on different sites of the same DNA molecule or of different molecules, is possible by employing various DP/CP sets. The fluorescence signal is normally adjusted according to internal standards that are known to be either monomorphic or polymorphic.

The spot colour or the ratio of different colours, properly adjusted, determines if the DNA under investigation is mono-morphic or poly-morphic (Chessa S, Chiatti F, Ceriotti G, Caroli A, Consolandi C, Pagnacco G, Castiglioni B. Development of a single nucleotide polymorphism genotyping microarray platform for the identification of bovine milk protein genetic polymorphisms J Dairy Sci. (2007) 90: 451-64).

By way of example, when analysing a polymorphism, the CP will be common and each DP will represent a different allele, the CP probe will be ligated to one or another DP depending on the allele present on the target sequence. When both alleles are present in the DNA examined (i.e. the sample is heterozygous) CPs will be ligated alternatively to one or another DP, in a fashion proportional to the corresponding allele ratio in the target DNA. The ratio between the various signals emitted by the fluorophores used to label different DPs (e.g. allele 1 in green and allele 2 in red) allows to determine whether the analysed DNA is homozygous or heterozygous.

It is hence possible to detect different polymorphisms at more sites simultaneously by using different sets of DP/CP probes. Up to date, the LDR-UA assay is performed using direct labelling techniques, i.e. the DP is directly labelled and will (when ligated and consequently anchored on the UA) indicate the presence of a given allele based on the fluorescent colour detected.

Although being a very useful technique, there are certain practical problems in the interpretation of the data obtained by the LDR-UA as carried out as present that are linked to the manufacture, storage and use of the DP labelled probes.

It is to be also noted that the labelled DPs are highly expensive.

The problems that every person dealing with LDR-UA has to face are the following:

Each DP is labelled separately with a high cost for the researcher, and each DP labelled probe obtained will be the result of a more or less efficient process, thus emitting a signal that can be more or less strong depending on the effectiveness of the labelling procedure. Although standardised, the labelling procedure, as any laboratory protocols, will have a yield that depends also on random factors, thus rendering the interpretation of the results already slightly difficult. However, this problem can be and is somehow solved by adjusting the interpretation of the data obtained.

The DP-labelled probes are stored by freezing and, as known by all technical experts, each freeze and thaw passage affects the fluorophore effectiveness by decreasing its fluorescence. As this technique is normally and mainly used to diagnose/genotype several loci in parallel, in a multiplex fashion, in most cases, fluorophore-labelled probes synthesized at different dates are used, said fluorophores thus being at significantly different stages of age/decay. The age/decay of the fluorophore attached to the DP affects the strength of the signal. The result is that, in a double stained LDR-UA procedure, one of the two fluorophores can emit a very strong signal (e.g. green) and the other a very week one (e.g. red) with the result that the difference with green homozygous signal can be almost identical to the heterozygous signal (resulting mostly green instead of orange) due to the weakness of the red signal, leading to incorrect result/diagnosis.

Hence, normally used LDR-UA labelled DPs usually cause the problems summarized in the following points:
1) Fluorophores, such as the Cy group (e.g. Cy2, Cy3, Cy3.5, Cy5, Cy5.5, Cy7) and many other known fluorophores are easily damaged by thawing/freezing cycles and by light (M Nagel, F Richter, P Haring-Bolivar and H Kurz A functionalized THz sensor for marker-free DNA analysis Phys. Med. Biol. 48 (2003) 3625-3636). This might bring to an incorrect interpretation of the results when fluorophores of different ages are used simultaneously. For instance, in the genotyping experiments by LDR-UA, the adjustment according to the internal standard is based on the assumption that all fluorophores have comparable intensities. This is not the case if new and partially degraded fluorophore-labeled probes are used in the same Probe Mix (PM). A simple solution would be splitting the PM into single use aliquots. This is not always possible because of the impossibility to know *a priori* the composition of the PM: it often happens that each experiment requires a different mix of probes, or different probes, with differing age of synthesis, have to be added to an already existing PM.

Finally, the degradation of fluorophores can in some cases render the probe not usable.
2) The cost of direct labelling of each LDR-UA probe is equivalent to about one third of the total cost of the probes.
3) The nucleotide bound to the fluorophore can influence the amount of fluorescence. For instance Guanine is known as fluorescence quencher.

At present, the problem described above is partially solved by the skilled person by putting an extreme care on the storage procedure of the DP labelled probe, by trying to order all DP labelled probes at the same time. The LDR-UA operator will encounter difficulties in interpreting results obtained with probes of different degradation status. For instance, in case of detection of a polymorphism, a very strong difference in fluorescence intensities can lead to the misinterpretation of the experiment output, as the weakest signal might be mistaken for background signal.

The ambiguous experiment output in many cases demands a repetition of the experiment itself with freshly labelled probes.

It is to be noted that even in most datasheets provided by manufacturers of fluorophores or of labelled molecules, the avoidance of repeated freeze-thawing is highly recommended, this is to underline how the damage on the fluorophore due to freeze-thawing is a well known problem.

The most direct way of solving the problem above, would be to identify stabilisers for the fluorophores that would allow preserving the DP in time maintaining almost unaltered the fluorescence characteristics.

Another way to solve the problem would be to identify the most suitable and resistant fluorophores and to select the said fluorophores for labelling the DPprobes.

A prior art document that teaches an indirect labelling strategy based on the interaction between biotin bound to the LDR probe and streptavidine bound to the fluorophore, by in which the probe, modified with a biotin molecule, is indirectly labeled with a fluorophore- streptavidine conjugate (Agnishwar Girigoswami, Cheulhee Jung, Hyo Young Mun, Hyun Gyu Park PCR-free mutation detection of BRCA1 on a zip-code microarray using ligase chain reaction J. Biochem. Biophys. Methods 70 (2008) 897-902). This document teaches the biotin labelling as an alternative way of performing LDR-UA. The problems mentioned in the present description are absolutely not taken into account on the document mentioned above. In any case, the document cited not only does not refer to the problems mentioned in the present description, but also provides a protocol wherein said problems exist only partially as the biotin/streptavidine system offers the opportunity to use only one fluorophore in the system (i.e. only one color per time). As a consequence, to distinguish each allele of a locus more than one ZIP/cZIP is required.

Furthermore, the protocol proposed therein does not solve the expense problems as the DP oligo labeling with biotin is only slightly cheaper than the direct Cy3 or Cy5 labeling: the cost of biotin labeling is about 80% of the Cy3/Cy5 direct labeling and costs for the streptavidin/fluorophore molecule for the signal detection have to be added to the labeling costs.

The main problem that the present invention intends to solve, hence, is how to overcome the uneven signals due to the time and storage linked decay of the fluorophore thus obtaining homogeneous signals in the detection of the UA in LDR-UA reactions with one or more fluorophore.

### DETAILED DESCRIPTION OF THE INVENTION

The applicant solves the problems left open in the state of the art, by designing an indirect labelling technique suitable for LDR-UA hence providing a method and new "labelling tools" perfectly suitable for the LDR-UA technique.

The indirect labeling strategy herein described replaces the fluorophore at the 5' end of the DP, with an oligonucleotide sequence (herein named Universal Labeling Sequence, ULS). The indirect labeling is achieved with a reverse and complementary sequence labeled with at least one fluorophore molecule (cULS).

This strategy improves the approaches cited above for the following reason:
1) Identical labelling is guaranteed at all DPs, resulting in identical fluoresce in all LDRs in one assay. This helps avoiding bias due to diverse fluorescence of probes.
2) The labelling fluorophore can be changed at any time by the simple substitution of the cULS with an identical oligo bound to another fluorophore.
3) Costs are considerably lower for the indirect labelling system herein proposed than in the existing direct labelling state of the art. The estimated costs correspond to the 50% of the direct labelling costs
4) ULS and cULS are designed to obtain the maximum efficiency in terms of fluorescence, minimizing the effects caused by the nucleotide carrying the fluorophore.
5) The cULS-mediated labeling offers the chance to use many different colors. This is particularly important when genotyping experiments that require the use of different DPs labeled with different fluorophores are carried out.

### Hence the object of the present invention is:

Complementary universal Labeling Sequences (cULS) for LDR-UA wherein said labelling sequences are labelled with a fluorophore and recognise Universal Labelling Sequences (ULS) located on LDR-UA DPs;
a method of indirect labelling in LDR-UA procedures comprising the steps of:
- carrying out a standard LDR-UA process with a CP probe comprising a given cZIP code at the 3' end and one or more suitable DP comprising a ULS at the 5' end
- detecting the resulting signal on the Universal Array by hybridising said one or more DP comprising a ULS with one or more labelled cULS, said cULS being, each, labelled with a different fluorophore (i.e. each DP is bound to a specific ULS, each corresponding cULS is labelled with a fluorophore different from the one used for each other cULS):

a kit for carrying out indirectly labelled LDR-UA comprising at least one or more vials of a DP bound to an ULS oligo wherein each of said vials can contain a different DP bound to a different ULS, one or more vials of a cULS labelled oligo wherein each of said vials can contain a different cULS, each different cULS being directly labelled with a different fluorophore;
a kit for carrying out indirectly labelled LDR-UA comprising at least one or more vials of a directly labelled cULS oligo, the kit may contain vials with a different cULS each, and each cULS can also be subdivided in vials in which said cULS is directly labelled with a different fluorophore.

### DETAILED DESCRIPTION OF THE FIGURES

### Figure 1

Figure 1 exemplifies the state of the art on the LDR-UA technique:
**A.** LDR-UA experiment outline. The polimorphism localized at the 3' end of Discriminating Probe (DP) is detected. The probes/target DNA perfect match (indicated in a circle) allows the DP and Common Probe (CP) to be ligated together by the thermostable ligase.
   The DNA is first denaturated at 94°C and then the reaction is incubated at 65°C for the annealing and ligation of the probes. Denaturation/annealing/ligation cycles are repeated (typically 30 times) to allow the signal to be linearly amplified.
**B.** LDR product hybridisation on Universal Array (UA). The LDR product is captured by the corresponding Zip Code. The Cy3 fluorophore at the 5' end of the DP renders the relative spot visible.

### Figure 2.

Example of LDR-UA experiment aimed to genotype a site on the target DNA. In order to reveal wheatear at a given position there is an A or a C nucleotide, two discriminating probes (DP-A and DP-C) are used. DP-A and DP-C are identical apart from the discriminating base (A in the first case and C in the second case) and the fluorophore they are associated to. Both DP can be ligated to the same CP if the proper target DNA is present. The colour of the spot will tell if only the A allele is present (green, Cy3), or only the C allele is present (red, Cy5) or both (yellow, Cy3+Cy5).

Figure 3 Indirect labeling of DP. The labeling occurs via a cULS that carries the fluorophore (i.e. Cy3) and anneals to the specific ULS positioned at the 5' end of the corresponding DP. Similarly, to assay for the presence of a different allele at the same locus, a different cULS/ULS set, labelled with a different fluorophore (i.e. Cy5), is used to the other DP.

Figure 4 represents the synthesis of a specific DP starting form a pre-prepared ULS

Figure 5 represents a reversed cZIP-CP-DP-ULS molecule wherein the ULS will be located at the 3' of the DP.

### DETAILED DESCRIPTION OF THE INVENTION

According to the present description, **LDR-UA** means Ligation Detection Reaction-Universal Array as the technique known in the art with said definition wherein a Common Probe (CP) ligated to a specific cZip code and a Determining Probe (DP) coding for a specific single nucleotide plymorphism or point mutation is hybridised to the target DNA to be analysed and in case of perfect match the two probes are ligated by a thermostable ligase. The CP-DP compex thus obtained is hence anchored on a universal microarray thanks to the cZIPcode bound on the CP and the results of the reaction are examined thanks to the use of fluorophores allowing the detection of the DP bound to the CP.

**CP** means Common Probe for LDR-UA, i.e. a probe comprising a region adjacent to the 3' of a mutation or a polymorphism that can be represented by a single nucleotide. Unless explicitly expressed the use of the term CP herein will indicate a Common Probe for LDR-UA phosphorilated at the 5' end and extended at its 3' end with a cZIP code for universal array as known in the art.

**DP** means Discriminating Probe for LDR-UA, i.e. a probe comprising a sequence which is the continuation of the 5' term of the CP, consisting, hence, at its 3' of the single point mutation or single nucleotide polymorphism under exam and the naturally following nucleotides, complementary to the target sequence, according to the present description, the 5' end of the DP is bound to a fluorophore when citing the prior art and bound to specific sequences when citing the invention, namely, the ULS defined herein below. The DP, hence, is the probe carrying the discriminating base.

**ULS** means Universal Labelling Sequence, according to the description, the USL is a kind of sequence that is selected in order to be suitable for binding at the 5' end of the DP for LDR-UA, to allow indirect labelling of the CP-DP complex by hybridisation with an labelled complementary sequence, hence, **cULS** is a sequence complementary to the ULS, directly labelled with a fluorophore, ready to use for the detection of the LDR-UA results without further detection passages (such as biotin/sptreptavidin FITC binding steps).

**Fluorophore** is a component of a molecule which causes a molecule to be fluorescent. It is a functional group in a molecule which will absorb energy of a specific wavelength and re-emit energy at a different (but equally specific) wavelength. The amount and wavelength of the emitted energy depend on both the fluorophore and the chemical environment of the fluorophore.

When the phrase "a sequence, a probe, an oligonucleotide, is **directly labelled** with a fluorophore", it is intended that the fluorophore is located on that molecule hence that the thus labelled sequence, probe, oligonucleotide emits fluorescence without further treatments as opposed to sequences, probes, oligonucleotides carrying a molecule that will react with a further molecule bound to the fluorophore such as a biotinylated nucleic acid that, in order to emit fluorescence, will have to react and to bind with a fluorophore-streptavindin complex.

One of the embodiments of the invention is hence represented by complementary Universal Labeling Sequences (cULS) for LDR-UA wherein said labeling sequences are directly labelled with a fluorophore and recognise Universal Labelling Sequences (ULS) located on LDR-UA DPs.

According to the description, the ULS represent the 5' extension of the DP instead of the fluorophore used in the art, and is selected in order to not cross hybridise with the DNA of the organism of interest and to not produce hairpin loops that would block the hybridising process with the corresponding cULS.

The cULS of the invention can be labelled with a fluorophore at one or both ends, or internally or the labelling can be at one or both ends and internally the double or multiple labelling would have the further advantage, with respect to the prior art, to increase the signal strength (namely to double it or more) and to facilitate the reading of the results obtained.

The immediate advantages offered by the ULS/cULS system of the invention is to avoid submitting the labelled molecule to all the passages of the LDR-UA technique limiting the introduction of the fluorophore which is known to be a delicate molecule (suffering also from heating cycles) at the last passages of the procedure. Furthermore, the system ULS/cULS herein described, allows the user to select the preferred dye at the last moment as a specific cULS can be provided, according to the invention, in several colours, i.e. bound to different fluorophores. This possibility allows the user to pick the most suitable fluorophore depending on the experiment to carry out. Moreover, the use of different ULS, allows to have a different corresponding ULS for each different colour, hence a multiple stained LDR-UA can be obtained (useful, i.e., when more than two alleles per locus are present).

One of the most important advantages is given by the fact that the cULS will be the same for all DP carrying the correspondent ULS rendering the results independent from differences in the age/decay of the DP-ULS used when more than one DP is used simultaneously. More specifically, it has to be kept in mind that different CP-DP sets, can be recognised by the colour and by their position on the array due to the fact that there are two markers, the cZIP probe that will position each specific CP in a specific position on the slide, and the ULS that will hybridise with the corresponding labelled cULS and will provide a specific colour/fluorescence.

With direct labelling of the DP, as mentioned before, a serious problem is caused by the difference in fluorescence of each labelled DP used simultaneously in the assay, due to age of the probe, storage conditions, frequency of use, differences in the rate of decay of each of the fluorophores used, etc. This in turn may lead to misinterpretations of the results as a heterozygous individual will present the same fluorescence intensities of a homozygous individual. The use of the same cULS to label all the corresponding ULS/DP in one assay will provide a homogeneous labelling for all the loci in the assay. This homogeneity in labelling will allow a more reliable interpretation of the results for all the loci in the assay. This will be true when using single colour assays (i.e. presence/absence of a sequence) versus double colour (i.e. genotyping of bi-allelic diploid loci) and even three or more colours (i.e. tri-allelic diploid loci or multi-allelic polyploid systems). The invention will bring more consistency to the results generated using the LDR-UA system.

The ULS in the invention could be, by way of example of SEQ ID 1,2, and the corresponding cULS (in the same order, so the first ULS mentioned corresponding to the first cULS mentioned, the second ULS to the second cULS and so on) could be, by way of example of SEQ IDs 1 and 2, 3 and 4, 5 and 6, 7 and 8, 9 and 10, 11 and 12, 13 and 14, 15 and 16, 17 and 18, 19 and 20, 21 and 22, 23 and 24, 25 and 26, 27 and 28, 29 and 30, 31 and 32, 33 and 34, 35 and 36, 37 and 38, 39 and 40, 41 and 42, 43 and 44, 45 and 46, 47 and 48, 49 and 50, 51 and 52, 53 and 54, 55 and 56, 57 and 58, 59 and 60, 61 and 62, 63 and 64, 65 and 66, 67 and 68, 69 and 70, 71 and 72, 73 and 74, 75 and 76, 77 and 78, 79 and 80, 81 and 82, 83 and 84, 85 and 86, 87 and 88, 89 and 90, 91 and 92, 93 and 94, 95 and 96, 97 and 98, 99 and 100, 101 and 102, 103 and 104 as indicated in the following Table 1.

**Table 1 suitable ULS and cULS pairs**

| | | | |
|---|---|---|---|
| ULS1 SEQ ID 2 | CGCCTCCCTGTCGTTCGCCCAC | cULS1 SEQ ID1 | AGTGGGCGAACGACAGGGAGGCG |
| ULS2 SEQ ID 4 | CCGATGCGATGCGAGCCGAGCG | cULS2 SEQ ID 3 | ACGCTCGGCTCGCATCGCATCGG |
| ULS3 SEQ ID 6 | GGTGAGCCCCGGAACGGTCAG | cULS3 SEQ ID 5 | CTGACCGTTCCGGGGCTCACC |
| ULS4 SEQ ID 8 | CCTGCCGATGCGCACCTGCTG | cULS4 SEQ ID 7 | CAGCAGGTGCGCATCGGCAGG |
| ULS5 SEQ ID 10 | CCGCTCCCCGCGGGGGTCAGC | cULS5 SEQ ID 9 | GCTGACCCCCGCGGGGAGCGG |
| ULS6 SEQ ID 12 | GCGGCCCGGTGCGGGCGCCCT | cULS6 SEQ ID 11 | AGGGCGCCCGCACCGGGCCGC |
| ULS7 SEQ ID 14 | GAACGGCCCATCCACGGCGAG | cULS7 SEQ ID 13 | CTCGCCGTGGATGGGCCGTTC |
| ULS8 SEQ ID 16 | GGCGACGTCGCGCGGCCGCGG | cULS8 SEQ ID 15 | CCGCGGCCGCGCGACGTCGCC |
| ULS9 SEQ ID18 | CGGAGCGGGGCCCGGCCGCCA | cULS9 SEQ ID 17 | TGGCGGCCGGGCCCCGCTCCG |
| ULS10 SEQ ID 20 | GAAGCTCGCCGATATAAGCAA | cULS10 SEQ ID 19 | TTGCTTATATCGGCGAGCTTC |
| ULS11 SEQ ID 22 | CGAGCCGGACCCTGTGCGGTG | cULS11 SEQ ID 21 | CACCGCACAGGGTCCGGCTCG |
| ULS12 SEQ ID 24 | CACGCCGTCTCCGACCCAGCT | cULS12 SEQ ID 23 | AGCTGGGTCGGAGACGGCGTG |
| ULS13 SEQ ID 26 | CTGCGGGGCTACGATCGTGGG | cULS13 SEQ ID 25 | CCCACGATCGTAGCCCCGCAG |
| ULS14 SEQ ID 28 | GCCCGGCCTTGCGCCACCGCT | cULS14 SEQ ID 27 | AGCGGTGGCGCAAGGCCGGGC |
| ULS15 SEQ ID 30 | CCCCAGTGACGTGGGCGGACA | cULS15 SEQ ID 29 | TGTCCGCCCACGTCACTGGGG |
| ULS16 SEQ ID 32 | CTGGCCGTTCCGACACCCTCC | cULS16 SEQ ID 31 | GGAGGGTGTCGGAACGGCCAG |
| ULS17 SEQ ID 34 | GCCCGGCGTGCGTCGCTGGCC | cULS17 SEQ ID 33 | GGCCAGCGACGCACGCCGGGC |
| ULS18 SEQ ID 36 | GCACAGGTCGCCGTCTGGCCC | cULS18 SEQ ID 35 | GGGCCAGACGGCGACCTGTGC |
| ULS19 SEQ ID 38 | AGCGTGCCGCGGGGGGTGGGC | cULS19 SEQ ID 37 | GCCCACCCCCCGCGGCACGCT |
| ULS20 SEQ ID 40 | TCGCTCTTTCACCGGTCTGCC | cULS20 SEQ ID 39 | GGCAGACCGGTGAAAGAGCGA |
| ULS21 SEQ ID 42 | ACGACTGCGGGCAGTCGCCTC | cULS21 SEQ ID 41 | GAGGCGACTGCCCGCAGTCGT |
| ULS22 SEQ ID 44 | GGCCGACGGGCGCGCTTGGGG | cULS22 SEQ ID 43 | CCCCAAGCGCGCCCGTCGGCC |
| ULS23 SEQ ID 46 | CTGGCGGTCGCGGCGGGGGCG | cULS23 SEQ ID 45 | CGCCCCCGCCGCGACCGCCAG |
| ULS24 SEQ ID 48 | CTCCCCTGCCCTGCAGCTGCG | cULS24 SEQ ID 47 | CGCAGCTGCAGGGCAGGGGAG |
| ULS25 SEQ ID 50 | CGGCGGCGCGGCGCGCGGCGC | cULS25 SEQ ID 49 | GCGCCGCGCGCCGCGCCGCCG |
| ULS26 SEQ ID 52 | CCGATCCGCCACGGCGCCCAG | cULS26 SEQ ID 51 | CTGGGCGCCGTGGCGGATCGG |
| ULS27 SEQ ID54 | CCGGCGAACCGGGGAAGGCGA | cULS27 SEQ ID 53 | TCGCCTTCCCCGGTTCGCCGG |
| ULS28 SEQ ID56 | ACCTGCGCGCGCCGTGTGCCG | cULS28 SEQ ID 55 | CGGCACACGGCGCGCGCAGGT |
| ULS29 SEQ ID 58 | CCCCCCGGCGGTCCTTGCCCC | cULS29 SEQ ID 57 | GGGGCAAGGACCGCCGGGGGG |
| ULS30 SEQ ID60 | GCGCCGGACCGCCCACCCCGG | cULS30 SEQ ID 59 | CCGGGGTGGGCGGTCCGGCGC |
| ULS31 SEQ ID 62 | GCACGGGCCCCAAGGGAGCGA | cULS31 SEQ ID 61 | TCGCTCCCTTGGGGCCCGTGC |
| ULS32 SEQ ID 64 | GCCCCGCGGGCGCGGTCGGCT | cULS32 SEQ ID 63 | AGCCGACCGCGCCCGCGGGGC |
| ULS33 SEQ ID 66 | CGGCGCGGCGAGCCGGGGGCA | cULS33 SEQ ID 65 | TGCCCCCGGCTCGCCGCGCCG |
| ULS34 SEQ ID 68 | GGCCCGCCGGCGGTACCCCGT | cULS34 SEQ ID 67 | ACGGGGTACCGCCGGCGGGCC |
| ULS35 SEQ ID 70 | GGGGGAAGCTCGCGCTGGCAG | cULS35 SEQ ID 69 | CTGCCAGCGCGAGCTTCCCCC |
| ULS36 SEQ ID 72 | CCGCGGAGCGCCGGGCCCGGG | cULS36 SEQ ID 71 | CCCGGGCCCGGCGCTCCGCGG |
| ULS37 SEQ ID 74 | GGGCCGCCGACGCCCCCACGT | cULS37 SEQ ID 73 | ACGTGGGGGCGTCGGCGGCCC |
| ULS38 SEQ ID 76 | CTGCACGGCCCGGGCACGGGC | cULS38 SEQ ID 75 | GCCCGTGCCCGGGCCGTGCAG |
| ULS39 SEQ ID 78 | GCGGCCGACCCGTGGCCCTAC | cULS39 SEQ ID 77 | GTAGGGCCACGGGTCGGCCGC |
| ULS40 SEQ ID 80 | GCGAACGACCTCCCCCAAGCT | cULS40 SEQ ID 79 | AGCTTGGGGGAGGTCGTTCGC |
| ULS41 SEQ ID 82 | AGTCTCTCACCAGGCTCACCA | cULS41 SEQ ID 81 | TGGTGAGCCTGGTGAGAGACT |
| ULS42 SEQ ID 84 | ACGGTTACCCGAGGCGGGTCC | cULS42 SEQ ID 83 | GGACCCGCCTCGGGTAACCGT |
| ULS43 SEQ ID 86 | GGCGGCCGCGCGCCTGGCCGC | cULS43 SEQ ID 85 | GCGGCCAGGCGCGCGGCCGCC |
| ULS44 SEQ ID 88 | TTGGACCCGTGATGAGCCGGG | cULS44 SEQ ID 87 | CCCGGCTCATCACGGGTCCAA |
| ULS45 SEQ ID 90 | TGGAGGCCGAGCCACGAACGA | cULS45 SEQ ID 89 | TCGTTCGTGGCTCGGCCTCCA |
| ULS46 SEQ ID 92 | CGCCCGCCTGGCCCCGAACGC | cULS46 SEQ ID 91 | GCGTTCGGGGCCAGGCGGGCG |
| ULS47 SEQ ID 94 | GGGACACACGGACGCCCCGGC | cULS47 SEQ ID 93 | GCCGGGGCGTCCGTGTGTCCC |
| ULS48 SEQ ID 96 | TTCCCGGGCCCCTGGGAGGGT | cULS48 SEQ ID 95 | ACCCTCCCAGGGGCCCGGGAA |
| ULS49 SEQ ID 98 | CCCGCCGGCAGGCCCTGAGGC | cULS49 SEQ ID 97 | GCCTCAGGGCCTGCCGGCGGG |
| ULS50 SEQ ID 100 | TGCTGGCCGGCTAGCATGCGC | cULS50 SEQ ID 99 | GCGCATGCTAGCCGGCCAGCA |
| ULS51 SEQ ID 102 | CCGCCACCCCCCCCCGAGGGG | cULS51 SEQ ID 101 | CCCCTCGGGGGGGGGTGGCGG |
| ULS52 SEQ ID 104 | GCCCACATGGTTCACCGCCGG | cULS52 SEQ ID103 | CCGGCGGTGAACCATGTGGGC |

In order to exemplify the carrying out of the invention a small example of the possible preparation of the ULS/cULS pairs for a double staining is showed below.
cULS-Cy3 5' Cy3-AGTGGGCGAACGACAGGGAGGCG 3' (i.e. Cy3-Seq ID 1)
cULS-Cy5 5' Cy5-ACGCTCGGCTCGCATCGCATCGG 3'(i.e. Cy5-Seq ID 3)
The corresponding ULS positioned at the 5' end of the DP are:
5' CGCCTCCCTGTCGTTCGCCCAC-Discriminating Probe 3' (i.e. Seq ID 2-DP)
5' CCGATGCGATGCGAGCCGAGCG-Discriminating Probe 3' (i.e. Seq ID 4-DP

The ULS according to the description can also be in a form suitable for further elongation in synthesis with the desired DP.

Synthesis techniques are known in the art, the 5' of the sequence to synthesise being often anchored in a suitable way. According to the description, the USL of the invention can be in a form suitable for further elongation at the 3' with the desired DP sequence.

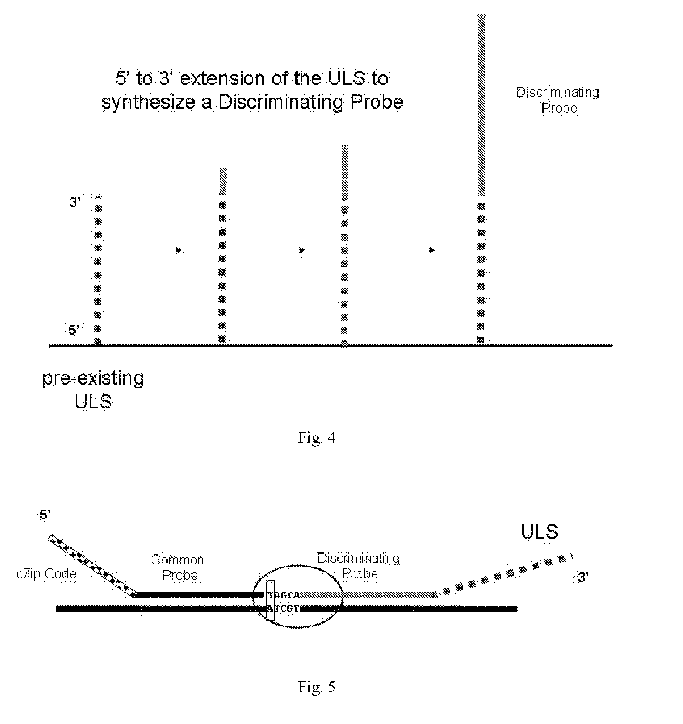

The extension of a pre-existing ULS with a DP is possible with a 5' to 3' oligo synthesis although it's less efficient than the more used 3' to 5' one (see figure 4). Alternatively it is possible to use the 3' to 5' synthesis but the DP-ULS and cZIP-CP molecules should be swapped (as represented in figure 5). In this case it must be considered that the discriminatory power of the system (that remains overall valid and suitable for genotyping) is decreased as the 5' end of the DP, where the investigated polymorphism is located, is less sensitive to mismatches.

According to the description the cULS can be directly labelled with any suitable fluorophore, systems requiring further passages besides the hybridisation of the ULS with the cULS are not envisaged. By way of example said further passages could be biotin/avidin binding , digoxigenin/rhodamin binding; the avoidance of said colouring systems is due to the fact that they have the further disadvantage of a weaker signal (not all molecules will bind) and of often providing a higher background due to the high "avidity" of the proteins bound to the fluorophore. Every skilled artisan is well aware of the background that can be often present on slides submitted to a detection procedure with the biotin/streptavidin-Fluorochrome system and similar systems. Hence, the cULS of the invention, can be labelled, as already mentioned at the 5' end, at the 3' end or both but the fluorophore used will be only a fluorophore directly emitting fluorescence, such as, by way of example but not limited to it, the Cy fluorophores (Cy2, Cy3, Cy3.5, Cy5, Cy5.5, Cy7), Fluorescein, 6-Fam, Hex, Tet, Tamra, Joe, Rox, IRDyeTM700, IRDyeTM800, Dyomics Dyes, Atto Dyes. Moreover, the cULS herein described could be labelled internally (hence not at their ends) or at one or both ends or even at one or both ends and internally. The cULS herein described can hence be labelled in order to emit a very strong signal, this would be advantageous when the polymorphism to detect is expected to emit a very weak signal or does emit a very weak signal.

The cULS herein described offers the advantage of being adaptable in order to emit a particularly intense signal if required.

The method of indirect labelling in LDR-UA procedures herein described requires the carrying out a standard LDR-UA process wherein one or more suitable CP probe comprises a given cZIP code at the 3' end and one or more suitable DP comprises a ULS at the 5' end and wherein the detection of the signal on the Universal Array is carried out by hybridising the thus obtained cZIP-CP-DP-ULS complex with one or more labelled corresponding cULS.

According to the present description, the ULS used in the same process can be more than one, in that case, they will be associated to different DPs, and the corresponding cULS will be, each, labelled with a different fluorophore. The fluorophores and the ULS/cULS pairs according to the present description will be as defined above. The advantages of the method of the invention are already discussed above when discussing the advantages of the ULS/cULS of the invention.

The LDR is carried out as described by Gerry et al. (1999). cULS are added to the hybridization mix after the reaction. The concentration of cULS is about ten times the concentration of the DP. cULS anneal to the corresponding ULS, resulting in the labelling of the probe (Figure 3).

According to the description, the method requires an LDR reaction as disclosed in the art and different CP-DP complexes can be examined in order to analyse different loci, different alleles or different mutations. The recognition of the sequence analysed is obtained by the position on the array (due to the Zip code-cZip code system) and by the ULS attached to the DP. Within each probe set (DPs-CP) Each different DP will be elongated with a specific ULS in order to allow a differential staining of each. As all cULS can be coloured with each of the suitable fluorophores, the person carrying out the method of the invention will be able to select the desired colour for each DP. The hybridisation step of the method, allowing the binding between ULS and the corresponding cULS can be carried out at the same time as the hybridisation step to allow the binding of the Zip code with the corresponding cZip code. This would have the advantage of not adding further cycles to the reaction but merely to add further reagents (i.e. the labelled cULS) to an already existing step thus not changing at all the backbone of the protocol (i.e. cycles, time, temperature)

The cULS can be added to the LDR mix before the reaction starts or immediately after the reaction finishes, as well as just before the hybridization step. The mix composed of LDR products, the cULS and the hybridation buffer is denatured at 94°C for 3' and immediately incubated in ice.

The method of the invention is suitable for carrying out genotyping analysis and single-base mutation diagnosis in humans, animals, plants, bacteria. In general it is suitable for the detection of nucleotide substitution, that can be represented by a single nucleotide, in all DNA sequence extracted from any matrix. Also, this could be employed in LOH studies, and perhaps in presence/absence assays for detection of pathogens.

Another object of the invention is a kit for carrying out indirectly labelled LDR-UA comprising at least one or more vials of a DP bound to an ULS oligo wherein each of said vials can contain a different DP bound to a different ULS and one or more vials of a cULS oligo directly labelled with a fluorophore wherein each of said vials can contain a different cULS.

The kit herein described, hence, can be suitable for specific diagnosis and contain DPs suitable for said diagnosis as known in the art, with the difference that the DP is elongated at 5' with a ULS according to the description. For multiple detections (i.e. a multiple diagnosis could be foreseen either for a group of diseases that would be convenient to diagnose together or for heterogeneous diseases wherein more mutations of the same gene and/or more mutations on more than one gene lead to the same phenotype, such as, by way of example, the hypertrophic cardiomyopathy and many other diseases known in the art).

The selection of the CPs and DPs would be evident from the known mutation site and the skilled person would have no problems whatsoever in determining said sequences, many of them being already known in the art. In case of diagnostic kits, the kit could also comprise the suitable CP probe bound to a universal cZIPcode.

The kit applies also for genotyping mutations that could be, again, on known loci for which LDR is already carried out or the manufacturer could provide the kit to the client with the CPs and DPs desired by the client. Of course, the kit could be limited to the DP and the ULS and the cULS system could be sold separately, the cULS system, by way of example, being composed by a stock of differently labelled specific cULS (i.e. each cULS labelled with a different fluorophore or each cULS provided in sub-vials comprising the said cULS labelled with a different fluorophore thus providing each cULS in several colours).

Alternatively, the kit could comprise one or more ULS suitable for elongation at the 3' allowing the user to order the synthesis of the ULS-DP complex where preferred and at the time preferred, together with one or more corresponding cULS directly labelled.

Both kits, can comprise, in terms of cULS the following:
one or more vials of a single cULS labelled with one fluorophore, one or more vials of the same cULS wherein each vial or group of vials is labelled with a different fluorophore, allowing hence a "set of colours" for a single cULS (i.e. cULS "a" in green, yellow, blue etc). The kit can also comprise more than one cULS (each one separately, ready to mix) of a different colour (es cULS "a" in red, cULS "b" in green, cULS "c" in blue etc.) or each different cUSL could still be provided in more than one colour allowing the user to decide which preferred and more suitable colour combination to use for each experiment, or even to try different colour combinations in order to select the better one for any given experiment or protocol. The skilled person knows well that, depending on what is under analysis, it is sometimes preferable to have a different colour for a given probe.

The kit of the invention hence allows large cross-possibilities to the user.

The following examples are described a mode to carry out the invention without limiting the invention to the mode described therein.

### EXAMPLES

### Indirect labelling of LDR-UA

Two polymorphic regions of ca. 1000bp each were amplified by PCR from genomic DNA extracted from cattle blood. Five probe sets were used to investigate five polymorphic sites on the two PCR fragments. Each set was composed of one CP and two DP, carrying different ULS and representing a different allele (A or B).

Genomic DNA used as template represented different known aplotypes. The investigated samples had different composition for each site (AA, AB or BB) apart from the reference site that was always polymorphic (AB).

The LDR was carried out in a volume of 20 µl. The reaction mix included: 1 unit of *Pfu* Ligase(Stratagene), 2 µl of 10x buffer, 10-100 ng of purified PCR product and CPs and DPs to the final concentration of 50 nM each.

The mix was first denatured for 10' at 94°C, followed by 30" at 94°C and 4' at 65°C, repeated for 30 cycles.

The reaction was then added to 45 µl hybridization buffer (1,10 M NaCl, 0,11 Sodium Citrate, 7 µg Salmon Sperm) and to cULS (500nM each). The solution was denatured at 94°C for 3' and then chilled on ice.

Samples were then hybridised on UA at 65°C for 2 hours. The UA was then washed in SSC 2X (0,3 M NaCl, 0,03M Sodium Citrate) and 0,1% SDS.

Hybridised UAs were scanned by a laser scanner Scanner (Perkin Elmer). The acquired image was then analysed to quantify the fluorescence intensity.

Signals from different channels were balanced according to values from reference sample. The reference samples are assumed to be perfectly polymorphic (with an AB configuration) and the ratio from the two colours should be 1:1. If this is not true (for difference in fluorophore efficiency, etc) the value of the ratio will serve to adjust all fluorescence values from the other DP. For instance if A:B in the reference sample is 0,75 means that the fluorophore B gives a stronger signal than the fluorophore A. Therefore all signals from fluorophore B must be reduced by a factor of 0.75.

Fluorescence values (from fluorophore A and B) from each spot are divided by the sum of the values of the two channels (A/A+B). Theoretically if the investigated DNA site is monomorphic the result is either 1 or 0 (depending if only A or B is present) if it is polymorphic the result is 0,5.

The adjustment of the channel ratio is performed according to the reference sample. In this example the reference sample gives a normalization factor of 0,75. To have the adjusted sample ratio is obtained by diving the A/A+B formula by 0,75.

In general the normalization formula is (A/A+B)/(Arf/Brf), where Arf and Brf are values from the reference sample.

Values included between 0,20 and 0,80 indicated a polymorphic site (AB), values smaller than 0,20 indicate the site is BB and values greater than 0,80 represent a BB.

## Claims

1. One or more pair of sequences consisting of a Universal Labeling Sequence and a complementary Universal Labelling Sequence directly labelled with a fluorophore at least at one or both ends or internally.

2. The one or more pair of sequences according to claim 1 wherein the Universal Labelling Sequence is located at the 5' of a Discriminating Probe for LDR-UA.

3. The one or more pair of sequences according to anyone of claims 1 or 2 wherein each complementary Universal Labelling Sequence is labelled with a fluorophore selected from the group consisting of Cy2, Cy3, Cy3.5, Cy5, Cy5.5, Cy7, Fluorescein, 6-Fam, Hex, Tet, Tamra, Joe, Rox, IRDyeTM700, IRDyeTM800, Dyomics Dyes, Atto Dyes.

4. The one or more pair of sequences according to anyone of claims form 1 to 3, selected from the group consisting of: SEQ IDs 1 and 2, 3 and 4, 5 and 6, 7 and 8, 9 and 10, 11 and 12, 13 and 14, 15 and 16, 17 and 18, 19 and 20, 21 and 22, 23 and 24, 25 and 26, 27 and 28, 29 and 30, 31 and 32, 33 and 34, 35 and 36, 37 and 38, 39 and 40, 41 and 42, 43 and 44, 45 and 46, 47 and 48, 49 and 50, 51 and 52, 53 and 54, 55 and 56, 57 and 58, 59 and 60, 61 and 62, 63 and 64, 65 and 66, 67 and 68, 69 and 70, 71 and 72, 73 and 74, 75 and 76, 77 and 78, 79 and 80, 81 and 82, 83 and 84, 85 and 86, 87 and 88, 89 and 90, 91 and 92, 93 and 94, 95 and 96, 97 and 98, 99 and 100, 101 and 102, 13 and 104.

5. A method of indirect labelling of LDR-UA wherein a LDR-UA reaction is carried out with one or more cZip code-Common Probe complexes and one or more suitable Discriminating Probe comprising a Universal Labelling Sequence at the 5' end obtaining a 5'-Universal Labelling Sequence-Discriminating Probe-Common Probe-cZIP-3' complex, said LDR-UA reaction comprising the step of detecting the signal on the Universal Array by hybridising the 5'-Universal Labelling Sequence-Discriminating Probe-Common Probe-cZIP-3' complex thus obtained with one or more labelled corresponding complementary Universal Labelling Sequence and wherein each different complementary Universal Labelling Sequence is directly labelled with a different fluorochrome.

6. A kit for carrying out indirectly labelled LDR-UA comprising at least one or more vials of a Discriminating Probe bound to an Universal Labelling Sequence oligo wherein each of said vials can contain a different Discriminating Probe bound to a different Universal Labelling Sequence and one or more vials of a complementary Universal Labelling Sequence directly labelled oligo wherein each of said vials can contain a different complementary Universal Labelling Sequence and wherein each different complementary Universal Labelling Sequence is at least provided directly labelled with a different fluorophore.

7. The kit according to claim 6, wherein each different complementary Universal Labelling Sequence can be divided in different vials each different vial comprising said complementary Universal Labelling Sequence directly labelled with a different fluorophore.


SEQUENCE LISTING

<110>  Fondazione Parco Tecnologico Padano

<120>  METHOD FOR UNIFORMLY LABELLED LIGATION DETECTION REACTIONS (LDRs)
       ASSOCIATED TO UNIVERSAL ARRAYS AND LABELLED PROBES THUS OBTAINED

<130>  BE860R

<160>  104

<170>  PatentIn version 3.3

<210>  1
<211>  23
<212>  DNA
<213>  Artificial

<220>
<223>  cULS 1

<400>  1
agtgggcgaa cgacagggag gcg                                              23


<210>  2
<211>  22
<212>  DNA
<213>  Artificial

<220>
<223>  ULS1

<400>  2
cgcctccctg tcgttcgccc ac                                               22


<210>  3
<211>  23
<212>  DNA
<213>  Artificial

<220>
<223>  cULS2

<400>  3
acgctcggct cgcatcgcat cgg                                              23


<210>  4
<211>  22
<212>  DNA
<213>  Artificial

<220>
<223>  ULS2

<400>  4
ccgatgcgat gcgagccgag cg                                               22


<210>  5
<211>  21
<212>  DNA
<213>  Artificial

<220>
<223>  cULS3

<400>  5
ctgaccgttc cggggctcac c                                                21

<210>  6
<211>  21
<212>  DNA
<213>  Artificial

<220>
<223>  ULS3

<400>  6
ggtgagcccc ggaacggtca g                                                21


<210>  7
<211>  21
<212>  DNA
<213>  Artificial

<220>
<223>  cULS4

<400>  7
cagcaggtgc gcatcggcag g                                                21


<210>  8
<211>  21
<212>  DNA
<213>  Artificial

<220>
<223>  ULS4

<400>  8
cctgccgatg cgcacctgct g                                                21


<210>  9
<211>  21
<212>  DNA
<213>  Artificial

<220>
<223>  cULS5

<400>  9
gctgaccccc gcggggagcg g                                                21


<210>  10
<211>  21
<212>  DNA
<213>  Artificial

<220>
<223>  ULS5

<400>  10
ccgctccccg cgggggtcag c                                                21


<210>  11
<211>  21
<212>  DNA
<213>  Artificial

<220>
<223>  cULS6

<400>  11
agggcgcccg caccgggccg c                                                21

<210>  12
<211>  21
<212>  DNA
<213>  Artificial

<220>
<223>  ULS6

<400>  12
gcggcccggt gcgggcgccc t                                                21


<210>  13
<211>  21
<212>  DNA
<213>  Artificial

<220>
<223>  cULS7

<400>  13
ctcgccgtgg atgggccgtt c                                                21


<210>  14
<211>  21
<212>  DNA
<213>  Artificial

<220>
<223>  ULS7

<400>  14
gaacggccca tccacggcga g                                                21


<210>  15
<211>  21
<212>  DNA
<213>  Artificial

<220>
<223>  cULS8

<400>  15
ccgcggccgc gcgacgtcgc c                                                21


<210>  16
<211>  21
<212>  DNA
<213>  Artificial

<220>
<223>  ULS8

<400>  16
ggcgacgtcg cgcggccgcg g                                                21


<210>  17
<211>  21
<212>  DNA
<213>  Artificial

<220>
<223>  cULS9

<400>  17
tggcggccgg gccccgctcc g                                                21

<210>  18
<211>  21
<212>  DNA
<213>  Artificial

<220>
<223>  ULS9

<400>  18
cggagcgggg cccggccgcc a                                                21


<210>  19
<211>  21
<212>  DNA
<213>  Artificial

<220>
<223>  cULS10

<400>  19
ttgcttatat cggcgagctt c                                                21


<210>  20
<211>  21
<212>  DNA
<213>  Artificial

<220>
<223>  ULS10

<400>  20
gaagctcgcc gatataagca a                                                21


<210>  21
<211>  21
<212>  DNA
<213>  Artificial

<220>
<223>  cULS11

<400>  21
caccgcacag ggtccggctc g                                                21


<210>  22
<211>  21
<212>  DNA
<213>  Artificial

<220>
<223>  ULS11

<400>  22
cgagccggac cctgtgcggt g                                                21


<210>  23
<211>  21
<212>  DNA
<213>  Artificial

<220>
<223>  cULS12

<400>  23
agctgggtcg gagacggcgt g                                                21

<210>  24
<211>  21
<212>  DNA
<213>  Artificial

<220>
<223>  ULS12

<400>  24
cacgccgtct ccgacccagc t                                                21


<210>  25
<211>  21
<212>  DNA
<213>  Artificial

<220>
<223>  cULS13

<400>  25
cccacgatcg tagccccgca g                                                21


<210>  26
<211>  21
<212>  DNA
<213>  Artificial

<220>
<223>  ULS13

<400>  26
ctgcggggct acgatcgtgg g                                                21


<210>  27
<211>  21
<212>  DNA
<213>  Artificial

<220>
<223>  cULS14

<400>  27
agcggtggcg caaggccggg c                                                21


<210>  28
<211>  21
<212>  DNA
<213>  Artificial

<220>
<223>  ULS14

<400>  28
gcccggcctt gcgccaccgc t                                                21


<210>  29
<211>  21
<212>  DNA
<213>  Artificial

<220>
<223>  cULS15

<400>  29
tgtccgccca cgtcactggg g                                                21

<210>  30
<211>  21
<212>  DNA
<213>  Artificial

<220>
<223>  ULS15

<400>  30
ccccagtgac gtgggcggac a                                                21


<210>  31
<211>  21
<212>  DNA
<213>  Artificial

<220>
<223>  cULS16

<400>  31
ggagggtgtc ggaacggcca g                                                21


<210>  32
<211>  21
<212>  DNA
<213>  Artificial

<220>
<223>  ULS16

<400>  32
ctggccgttc cgacaccctc c                                                21


<210>  33
<211>  21
<212>  DNA
<213>  Artificial

<220>
<223>  cULS17

<400>  33
ggccagcgac gcacgccggg c                                                21


<210>  34
<211>  21
<212>  DNA
<213>  Artificial

<220>
<223>  ULS17

<400>  34
gcccggcgtg cgtcgctggc c                                                21


<210>  35
<211>  21
<212>  DNA
<213>  Artificial

<220>
<223>  cULS18

<400>  35
gggccagacg gcgacctgtg c                                                21

<210>  36
<211>  21
<212>  DNA
<213>  Artificial

<220>
<223>  ULS18

<400>  36
gcacaggtcg ccgtctggcc c                                                21


<210>  37
<211>  21
<212>  DNA
<213>  Artificial

<220>
<223>  cULS19

<400>  37
gcccaccccc cgcggcacgc t                                                21


<210>  38
<211>  21
<212>  DNA
<213>  Artificial

<220>
<223>  ULS19

<400>  38
   agcgtgccgc gggggtgggc c                                                 21


<210>  39
<211>  21
<212>  DNA
<213>  Artificial

<220>
<223>  cULS10

<400>  39
ggcagaccgg tgaaagagcg a                                                21


<210>  40
<211>  21
<212>  DNA
<213>  Artificial

<220>
<223>  ULS20

<400>  40
tcgctctttc accggtctgc c                                                21


<210>  41
<211>  21
<212>  DNA
<213>  Artificial

<220>
<223>  cULS21

<400>  41
gaggcgactg cccgcagtcg t                                                21

<210>  42
<211>  21
<212>  DNA
<213>  Artificial

<220>
<223>  ULS21

<400>  42
acgactgcgg gcagtcgcct c                                                21


<210>  43
<211>  21
<212>  DNA
<213>  Artificial

<220>
<223>  cULS22

<400>  43
ccccaagcgc gcccgtcggc c                                                21


<210>  44
<211>  21
<212>  DNA
<213>  Artificial

<220>
<223>  ULS22

<400>  44
ggccgacggg cgcgcttggg g                                                21


<210>  45
<211>  21
<212>  DNA
<213>  Artificial

<220>
<223>  cULS23

<400>  45
cgcccccgcc gcgaccgcca g                                                21


<210>  46
<211>  21
<212>  DNA
<213>  Artificial

<220>
<223>  ULS23

<400>  46
ctggcggtcg cggcgggggc g                                                21


<210>  47
<211>  21
<212>  DNA
<213>  Artificial

<220>
<223>  cULS24

<400>  47
cgcagctgca gggcagggga g                                                21

<210>  48
<211>  21
<212>  DNA
<213>  Artificial

<220>
<223>  ULS24

<400>  48
ctcccctgcc ctgcagctgc g                                                21


<210>  49
<211>  21
<212>  DNA
<213>  Artificial

<220>
<223>  cULS25

<400>  49
gcgccgcgcg ccgcgccgcc g                                                21


<210>  50
<211>  21
<212>  DNA
<213>  Artificial

<220>
<223>  ULS25

<400>  50
cggcggcgcg gcgcgcggcg c                                                21


<210>  51
<211>  21
<212>  DNA
<213>  Artificial

<220>
<223>  cULS26

<400>  51
ctgggcgccg tggcggatcg g                                                21


<210>  52
<211>  21
<212>  DNA
<213>  Artificial

<220>
<223>  ULS26

<400>  52
ccgatccgcc acggcgccca g                                                21


<210>  53
<211>  21
<212>  DNA
<213>  Artificial

<220>
<223>  cULS27

<400>  53
tcgccttccc cggttcgccg g                                                21

<210>  54
<211>  21
<212>  DNA
<213>  Artificial

<220>
<223>  ULS27

<400>  54
ccggcgaacc ggggaaggcg a                                                21


<210>  55
<211>  21
<212>  DNA
<213>  Artificial

<220>
<223>  cULS28

<400>  55
cggcacacgg cgcgcgcagg t                                                21


<210>  56
<211>  21
<212>  DNA
<213>  Artificial

<220>
<223>  ULS28

<400>  56
acctgcgcgc gccgtgtgcc g                                                21


<210>  57
<211>  21
<212>  DNA
<213>  Artificial

<220>
<223>  cULS29

<400>  57
ggggcaagga ccgccggggg g                                                21


<210>  58
<211>  21
<212>  DNA
<213>  Artificial

<220>
<223>  ULS29

<400>  58
ccccccggcg gtccttgccc c                                                21


<210>  59
<211>  21
<212>  DNA
<213>  Artificial

<220>
<223>  cULS30

<400>  59
ccggggtggg cggtccggcg c                                                21

<210>  60
<211>  21
<212>  DNA
<213>  Artificial

<220>
<223>  ULS30

<400>  60
gcgccggacc gcccaccccg g                                                21


<210>  61
<211>  21
<212>  DNA
<213>  Artificial

<220>
<223>  cULS31

<400>  61
tcgctccctt ggggcccgtg c                                                21


<210>  62
<211>  21
<212>  DNA
<213>  Artificial

<220>
<223>  ULS31

<400>  62
gcacgggccc caagggagcg a                                                21


<210>  63
<211>  21
<212>  DNA
<213>  Artificial

<220>
<223>  cULS32

<400>  63
agccgaccgc gcccgcgggg c                                                21


<210>  64
<211>  21
<212>  DNA
<213>  Artificial

<220>
<223>  ULS32

<400>  64
gccccgcggg cgcggtcggc t                                                21


<210>  65
<211>  21
<212>  DNA
<213>  Artificial

<220>
<223>  cULS33

<400>  65
tgcccccggc tcgccgcgcc g                                                21

<210>  66
<211>  21
<212>  DNA
<213>  Artificial

<220>
<223>  ULS33

<400>  66
cggcgcggcg agccgggggc a                                                21


<210>  67
<211>  21
<212>  DNA
<213>  Artificial

<220>
<223>  cULS34

<400>  67
acggggtacc gccggcgggc c                                                21


<210>  68
<211>  21
<212>  DNA
<213>  Artificial

<220>
<223>  ULS34

<400>  68
ggcccgccgg cggtaccccg t                                                21


<210>  69
<211>  21
<212>  DNA
<213>  Artificial

<220>
<223>  cULS35

<400>  69
ctgccagcgc gagcttcccc c                                                21


<210>  70
<211>  21
<212>  DNA
<213>  Artificial

<220>
<223>  ULS35

<400>  70
gggggaagct cgcgctggca g                                                21


<210>  71
<211>  21
<212>  DNA
<213>  Artificial

<220>
<223>  cULS36

<400>  71
cccgggcccg gcgctccgcg g                                                21

<210>  72
<211>  21
<212>  DNA
<213>  Artificial

<220>
<223>  ULS36

<400>  72
ccgcggagcg ccgggcccgg g                                                21


<210>  73
<211>  21
<212>  DNA
<213>  Artificial

<220>
<223>  cULS37

<400>  73
acgtgggggc gtcggcggcc c                                                21


<210>  74
<211>  21
<212>  DNA
<213>  Artificial

<220>
<223>  ULS37

<400>  74
gggccgccga cgcccccacg t                                                21


<210>  75
<211>  21
<212>  DNA
<213>  Artificial

<220>
<223>  cULS38

<400>  75
gcccgtgccc gggccgtgca g                                                21


<210>  76
<211>  21
<212>  DNA
<213>  Artificial

<220>
<223>  ULS38

<400>  76
ctgcacggcc cgggcacggg c                                                21


<210>  77
<211>  21
<212>  DNA
<213>  Artificial

<220>
<223>  cULS39

<400>  77
gtagggccac gggtcggccg c                                                21

<210>  78
<211>  21
<212>  DNA
<213>  Artificial

<220>
<223>  ULS39

<400>  78
gcggccgacc cgtggcccta c                                                21


<210>  79
<211>  21
<212>  DNA
<213>  Artificial

<220>
<223>  cULS40

<400>  79
agcttggggg aggtcgttcg c                                                21


<210>  80
<211>  21
<212>  DNA
<213>  Artificial

<220>
<223>  ULS40

<400>  80
gcgaacgacc tcccccaagc t                                                21


<210>  81
<211>  21
<212>  DNA
<213>  Artificial

<220>
<223>  cULS41

<400>  81
tggtgagcct ggtgagagac t                                                21


<210>  82
<211>  21
<212>  DNA
<213>  Artificial

<220>
<223>  ULS41

<400>  82
agtctctcac caggctcacc a                                                21


<210>  83
<211>  21
<212>  DNA
<213>  Artificial

<220>
<223>  cULS42

<400>  83
ggacccgcct cgggtaaccg t                                                21

<210>  84
<211>  21
<212>  DNA
<213>  Artificial

<220>
<223>  ULS42

<400>  84
acggttaccc gaggcgggtc c                                                21


<210>  85
<211>  21
<212>  DNA
<213>  Artificial

<220>
<223>  cULS43

<400>  85
gcggccaggc gcgcggccgc c                                                21


<210>  86
<211>  21
<212>  DNA
<213>  Artificial

<220>
<223>  ULS43

<400>  86
ggcggccgcg cgcctggccg c                                                21


<210>  87
<211>  21
<212>  DNA
<213>  Artificial

<220>
<223>  cULS44

<400>  87
cccggctcat cacgggtcca a                                                21


<210>  88
<211>  21
<212>  DNA
<213>  Artificial

<220>
<223>  ULS44

<400>  88
ttggacccgt gatgagccgg g                                                21


<210>  89
<211>  21
<212>  DNA
<213>  Artificial

<220>
<223>  cULS45

<400>  89
tcgttcgtgg ctcggcctcc a                                                21

<210>  90
<211>  21
<212>  DNA
<213>  Artificial

<220>
<223>  ULS45

<400>  90
tggaggccga gccacgaacg a                                                21


<210>  91
<211>  21
<212>  DNA
<213>  Artificial

<220>
<223>  cULS46

<400>  91
gcgttcgggg ccaggcgggc g                                                21


<210>  92
<211>  21
<212>  DNA
<213>  Artificial

<220>
<223>  ULS46

<400>  92
cgcccgcctg gccccgaacg c                                                21


<210>  93
<211>  21
<212>  DNA
<213>  Artificial

<220>
<223>  cULS47

<400>  93
gccggggcgt ccgtgtgtcc c                                                21


<210>  94
<211>  21
<212>  DNA
<213>  Artificial

<220>
<223>  ULS47

<400>  94
gggacacacg gacgccccgg c                                                21


<210>  95
<211>  21
<212>  DNA
<213>  Artificial

<220>
<223>  cULS48

<400>  95
accctcccag gggcccggga a                                                21

<210>  96
<211>  21
<212>  DNA
<213>  Artificial

<220>
<223>  ULS48

<400>  96
ttcccgggcc cctgggaggg t                                                21


<210>  97
<211>  21
<212>  DNA
<213>  Artificial

<220>
<223>  cULS49

<400>  97
gcctcagggc ctgccggcgg g                                                21


<210>  98
<211>  21
<212>  DNA
<213>  Artificial

<220>
<223>  cULS49

<400>  98
cccgccggca ggccctgagg c                                                21


<210>  99
<211>  21
<212>  DNA
<213>  Artificial

<220>
<223>  cULS50

<400>  99
gcgcatgcta gccggccagc a                                                21


<210>  100
<211>  21
<212>  DNA
<213>  Artificial

<220>
<223>  ULS50

<400>  100
tgctggccgg ctagcatgcg c                                                21


<210>  101
<211>  21
<212>  DNA
<213>  Artificial

<220>
<223>  cULS51

<400>  101
cccctcgggg gggggtggcg g                                                21

<210>  102
<211>  21
<212>  DNA
<213>  Artificial

<220>
<223>  ULS51

<400>  102
ccgccacccc cccccgaggg g                                                21


<210>  103
<211>  21
<212>  DNA
<213>  Artificial

<220>
<223>  cULS52

<400>  103
ccggcggtga accatgtggg c                                                21


<210>  104
<211>  21
<212>  DNA
<213>  Artificial

<220>
<223>  ULS52

<400>  104
gcccacatgg ttcaccgccg g                                                21